# EUROPEAN PATENT SPECIFICATION

(11) **EP 3 411 097 B1**
(45) Date of publication and mention of the grant of the patent: **25.11.2020**
(21) Application number: 16705639.9
(22) Date of filing: 02.02.2016
(51) Int. Cl.: A61M 5/142, A61M 5/145

(54) **HYBRID AMBULATORY INFUSION PUMP WITH CONTACTLESS POWER TRANSMISSION**
HYBRIDE AMBULANTE INFUSIONSPUMPE MIT KONTAKTLOSER ENERGIEÜBERTRAGUNG
POMPE DE PERFUSION AMBULATOIRE HYBRIDE AVEC TRANSMISSION D'ÉNERGIE SANS CONTACT

(43) Date of publication of application: 12.12.2018
(73) Proprietor: Perqflo, LLC, Valencia, CA 91355 (US)
(72) Inventor: SMITH, Roger, E., Ivins, UT 84738 (US)
(74) Representative: MERH-IP Matias Erny Reichl Hoffmann Patentanwälte PartG mbB
(86) International application number: PCT/US2016/016214
(87) International publication number: WO 2017/135936

(56) References cited:
- EP-A2- 2 324 872
- WO-A2-2009/113060
- CH-A5- 688 224
- US-A- 4 767 378
- US-A1- 2012 302 844
- US-A1- 2015 126 934

## Description

### BACKGROUND

### 1. Field

The present devices and methods relate generally to ambulatory infusion pumps.

### 2. Description of the Related Art

Ambulatory infusion pumps (also referred to herein simply as "infusion pumps") are relatively small, at least substantially self-contained devices that are used to introduce drugs and other infusible substances (collectively "medicament") into patients' bodies. Some infusion pumps are configured to be worn on a belt, carried in a clothing pocket, or the like. Other infusion pumps are configured to be adhered to skin in patch-like fashion. Infusion pumps are advantageous in that they may be used to, for example, subcutaneously introduce (or "infuse") medicament on an ongoing or even continuous basis outside of a clinical environment. Infusion pumps are also advantageous in that they greatly reduce the frequency of subcutaneous access events such as needle-based shots. One example of a medicament that may be introduced by an infusion pump is a liquid formulation of insulin. Other exemplary medicaments that may be introduced by an infusion pump include, but are not limited to, drugs that treat cancers and drugs that suppress the perception of pain.

US 2012/0302844 A1 shows a medical patch device which is mountable on a skin for drug delivery or analyte sensing.

CH 688 224 A5 shows an implantable apparatus for dosing pharmaceutical fluids into a human or an animal body.

WO 2009/113060 A2 shows a portable ambulatory fluid delivery device including a dispensing unit to dispense therapeutic fluid.

Many conventional infusion pumps have improved patient health and quality of life. Nevertheless, the present inventors have determined that conventional infusion pumps are susceptible to a wide range of improvements. By way of example, but not limitation, the present inventors have determined that it would be desirable to provide an infusion pump that is smaller, simpler, and less costly than conventional infusion pumps, while still being more accurate than conventional infusion pumps.

### SUMMARY

The invention is defined by the subject-matter of independent claims 1 and 11. Further embodiments are defined in the respective dependent claims.

A system in accordance with at least one of the present inventions includes an infusion pump durable assembly and an infusion pump disposable assembly. The durable assembly may include one or more energy sources in a a housing. The disposable assembly may include a medicament reservoir and be configured to attach to the durable assembly. Energy from the energy supply may be transferred to the disposable assembly to dispense medicament from the reservoir. The energy may be transferred without mechanical coupling or electrical contact, as by electromechanical torque coupling.

A method in accordance with at least one of the present inventions includes the step of securing an infusion pump durable assembly with a energy supply to an infusion pump disposable assembly with a medicament reservoir such that energy from the energy supply is transferred to the disposable assembly and transformed into mechanical forces that move a plunger within the reservoir.

A disposable assembly in accordance with at least one of the present inventions includes a baseplate, a medicament reservoir on the baseplate, a plunger moveable within the reservoir, a motor rotor, and a gear train. The motor rotor may receive electromagnetic energy from outside the disposable assembly and the gear train may transform that electromagnetic energy to mechanical forces that move the plunger within the reservoir.

The features and attendant advantages of the present inventions will become apparent as the inventions become better understood by reference to the following detailed description when considered in conjunction with the accompanying drawings.

### BRIEF DESCRIPTION OF THE DRAWINGS

Detailed description of exemplary embodiments will be made with reference to the accompanying drawings.
FIG. 1A is a perspective view of an exemplary infusion pump system in an assembled state.
FIG. 1B is an exploded perspective view of the infusion pump system illustrated in FIG. 1A, including a durable assembly and a disposable assembly.
FIG. 2 is a top view of certain components of the infusion pump system illustrated in FIGS. 1A and 1B.
FIGS. 2A is a schematic view showing a use of the infusion pump system illustrated in FIGS. 1A and 1B.
FIG. 2B is a schematic view showing another use of the infusion pump system illustrated in FIGS. 1A and 1B.
FIG. 3A is a perspective view of an exemplary durable assembly.
FIG. 3B is a perspective view of certain components of the durable assembly illustrated in FIG. 3A.
FIG. 4A is a perspective view of an exemplary disposable assembly.
FIG. 4B is a perspective view of certain components of the disposable assembly illustrated in FIG. 4A.
FIG. 5A is a perspective view of certain components of a durable assembly and a disposable assembly of an exemplary infusion pump system.
FIG. 5B is a perspective view of the components of the exemplary durable assembly illustrated in FIG. 5A.
FIG. 5C is a perspective view of the components of the exemplary disposable assembly illustrated in FIG. 5A.
FIG. 6 is a perspective section view of components of the exemplary infusion pump system of FIG. 5A, revealing a gap between certain components of the durable and disposable assemblies.
FIG. 7 is a front view showing a patient's skin being cleaned.
FIG. 8 is a flow chart illustrating an exemplary disposable assembly removal and replacement method.
FIG. 9 is a perspective view of an exemplary infusion pump system in an assembled state.

### DETAILED DESCRIPTION

The following is a detailed description of the best presently known modes of carrying out the inventions. This description is not to be taken in a limiting sense, but is made merely for the purpose of illustrating the general principles of the inventions.

It should also be noted here that the specification describes structures and methods that are especially well-suited for the subcutaneous delivery of high concentration insulin (i.e., U-200 insulin and above) such as U-500 insulin as well as lower concentration insulin such as U-100 insulin. Nevertheless, it should be appreciated that the present inventions are applicable to a wide variety of infusion pumps and medicaments. By way of example, but not limitation, the inventions may employ, for fluid displacement, a reservoir with a plunger, a fluid displacement device in the form of a plunger pusher, and a drive mechanism that includes a motor, or other fluid displacement devices, regardless of the type of reservoir employed, piston pumps (e.g., electromagnet pumps), MEMS pumps, peristaltic pumps and any other suitable pumps as well as corresponding drive mechanisms. Exemplary infusion pumps that include a reservoir with a plunger, a fluid displacement device in the form of a plunger pusher, and a drive mechanism are described in U.S. patent application serial number 12/890,207, filed September 24, 2010, and corresponding U.S. patent publication number 2012/0078170, both of which are incorporated by reference in their entireties. The present inventions are also applicable to medicaments such as, for example, drugs to mask pain, chemotherapy and other cancer related drugs, antibiotics, hormones, GLP-1, Glucagon, various other drugs that include large molecules and proteins that may require a high level of delivery accuracy, as well as to high concentration insulin (i.e., U-200 insulin and above) such as U-500 insulin and lower concentration insulin such as U-100 insulin.

As noted above, some ambulatory infusion pumps are intended to be worn on a belt, carried in a pocket, or otherwise supported within a holder of some kind (referred to collectively as "pocket pumps"). Such infusion pumps transfer fluid from a reservoir to an infusion set by way of an elongate tube. Subcutaneous access may be obtained by way of a cannula in the infusion set. Other ambulatory infusion pumps are intended to be adhered to the skin above the delivery site (sometimes referred to as "patch pumps"). Here, the cannula or other subcutaneous access device may extend directly from the infusion device. Given these modes of use, patients typically prefer the device to be as small as possible so it is more comfortable, less obtrusive, and less visible. In addition, patients want a device that is easy and convenient to use.

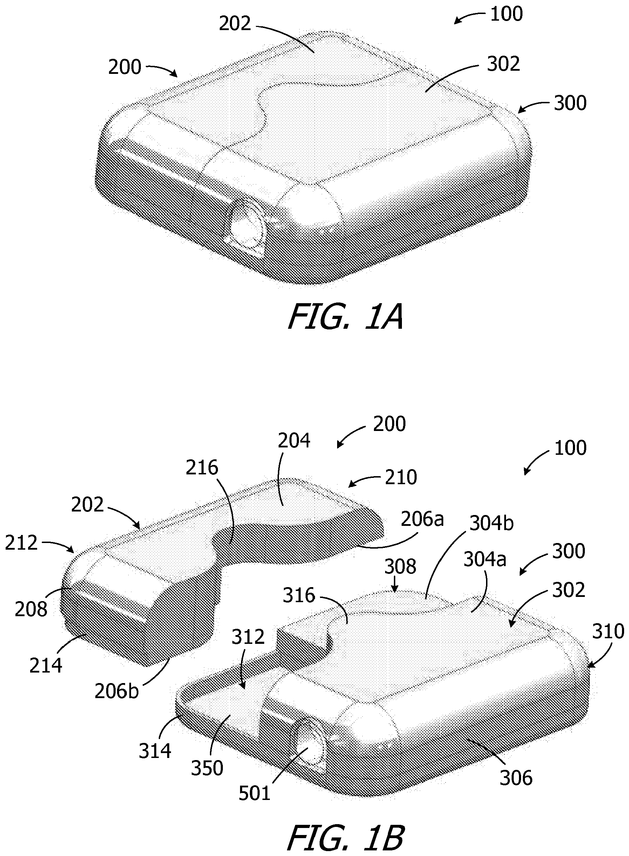

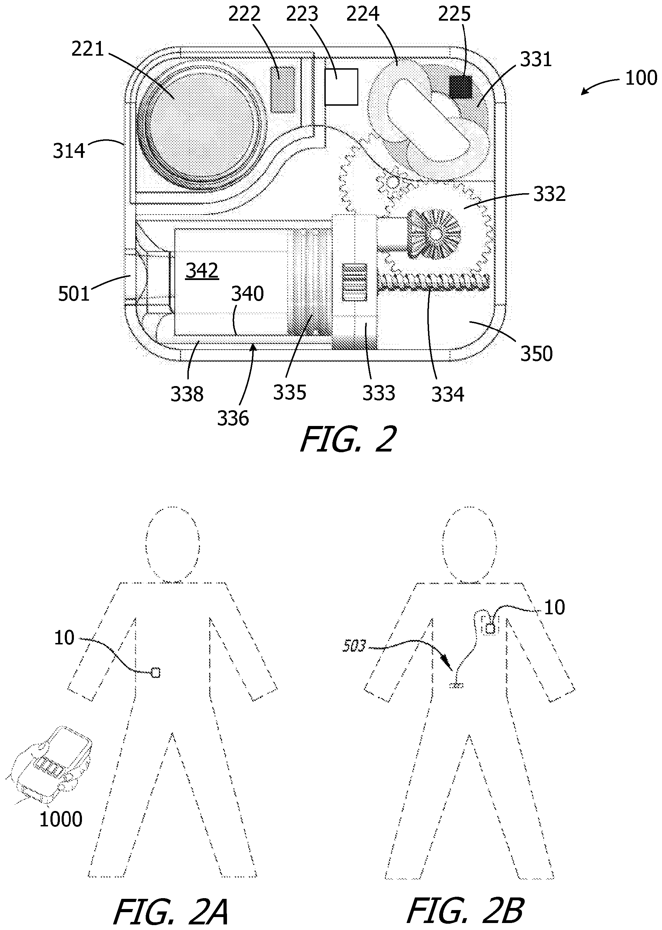

An exemplary ambulatory infusion system, which is generally represented by reference numeral 100 in FIGS. 1A, 1B, and 2, includes a durable assembly 200 and a disposable assembly 300. Exemplary durable assembly 200 includes a housing 202, one or more batteries or other energy supply 221, one or more capacitors or other energy storage 222, a microprocessor 223, a coil assembly 224 (which functions as a motor stator), and one or more Hall effect sensors 225. Exemplary disposable assembly 300 includes a baseplate 350 supporting components such as a magnetic motor rotor 331, a gear train 332 including lead screw drive gear 333, and a lead screw 334 attached to plunger 335, which is positioned in a medicament reservoir 336. A cover 302, under which some or all of the magnetic motor rotor 331, gear train 332 (with drive gear 333), lead screw 334, plunger 335, and medicament reservoir 336 are located in various embodiments, may be mounted to the baseplate 350.

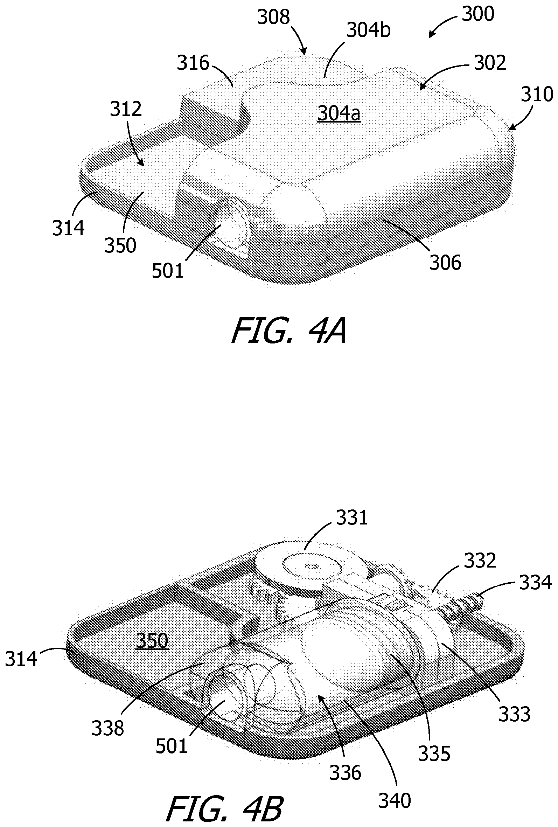

The exemplary disposable assembly 300 may be secured to the exemplary durable assembly 200, as shown in FIGS. 1A and 2. To that end, the exemplary housing 202 includes a top wall 204, bottom walls 206a and 206b and a side wall 208 that together define a relatively thin housing portion 210 and a relatively thick housing portion 212. An indentation 214 is formed in the relatively thick portion 212. The exemplary cover 302 includes top walls 304a and 304b and a side wall 306 that together define a relatively thin cover portion 308 and a relatively thick cover portion 310. A portion of the baseplate 350 is not covered by the cover 302, thereby defining a recess 312 that is bordered by a wall 314 that extends around the baseplate (see also FIG. 4B). When the durable and disposable assemblies 200 and 300 are secured to one another in the manner illustrated in FIG. 1A, the relatively thick portion 212 of the housing 202 will reside in the recess 312 of the disposable assembly 300 (with the wall 314 in the indentation 214). The relatively thin portion 210 of the housing 202 will reside on the top wall 304b of the cover 302. The cover 302 also includes a projection 316 that mates with a recess 216 on the housing 202. Additionally, as is discussed in greater detail below, the disposable assembly 300 may be configured for different medicaments, such as different medicament concentrations, different medicament amounts, or different modes of system operation.

In other implementations, the cover 302 may be configured to cover fewer than all of the components on the baseplate 350. For example, a cover may be configured such that the magnetic motor rotor 331 and a portion of the gear train 332 are not under the cover, while the remaining components are under the cover. In still other implementations, the cover 302 may be omitted and the durable assembly 200 may be configured to cover all of the components on the baseplate 350.

As discussed in U.S. patent publication number 2012/0078170 described above, and in U.S. application Serial No. 13/300,574, filed November 19, 2011, and corresponding U.S. patent publication number 2012/0184907, and in U.S. application Serial No. 13/475,843, filed May 18, 2012, and corresponding U.S. patent publication number 2013/0138078, each of which are incorporated by reference in their entireties, ambulatory infusion systems that employ a reservoir on a baseplate may be configured for different types of use. For example, disposable assembly 300 may be adhered to the patient's skin and may be used in conjunction with a cannula (not shown) that is operatively connected to the reservoir 336 so that the system 100 may be deployed as a "patch-pump," as shown in FIG. 2A. Alternatively, as shown in FIGS. 2B, the baseplate 350 of disposable assembly 300 may be configured to operably connect the reservoir 336 to an infusion set 503 (e.g., by way of the illustrated infusion set tube and a connector 501 shown in FIGS. 1B and 2) so that the system 100 may be deployed as a "pocket pump," a "belt-worn pump" or some other wearable pump. In other words, using the same durable assembly 200, the user may configure the system for use as "pocket pump" or a "patch pump" by simply selecting the appropriate disposable assembly and attaching the disposable assembly to the durable assembly. The user may also switch from one configuration to another, by simply removing one disposable assembly and replacing it with another disposable assembly.

It should therefore be noted that the present inventions include kits that contain various combinations of disposable assemblies, where at least two of the disposable assemblies may be different. Additionally or alternatively, kits or other packages may include various disposable assembly components, such as an infusion set and/or cannula inserter. Kits may also include a durable assembly. The disposable assemblies in such kits may also include the detection/identification instrumentalities discussed below. The components of the present kits (e.g., combination of various disposable assemblies and/or components) may be stored in a common package, with individual packages for each component if necessary, and provided to the user in the common package. Other components that may be provided in such kits include, but are not limited to, inserters that are preloaded with a cannula, and cleaning swabs. A recharger may also be provided in a kit that includes a durable assembly.

In addition to disposable assembly packaging and labeling, the different disposable assemblies may include visual cues to differentiate the various disposable assemblies. For instance, disposable assemblies with different concentrations of medicament or different medicament fill volumes may use different colors for the reservoir and/or baseplate of the disposable assembly, or mechanical features that ensure disposables are only able to attach to correctly programmed durables.

It should also be noted here that, but for the issue of priming, the dispensing procedures associated with an infusion system "patch pump" configuration, which may include a durable assembly 200 and a disposable assembly 300, are substantially the same as the dispensing procedures associated with a "pocket pump" configuration, which may also include an infusion set 503 (see FIGS. 2B). With a "patch pump" configuration, priming is not necessary because the volume of the associated cannula will be very small and there is a direct connection between the cannula and the medicament reservoir. Priming is, however, required to fill the infusion set tube (FIG. 2B) in a "pocket pump" configuration prior to the onset of medicament delivery. For instance, 20-30 µl may be required to fill the entire infusion set tube and, accordingly, the priming procedure may involve the rapid delivery of 10-15 IUs of U-500 insulin to the tube. The present inventors have determined that it would be advantageous to prevent users from initiating a priming procedure when the system is in the "patch pump" configuration, with a cannula positioned to deliver medicament essentially directly from the medicament reservoir to the patient, because rapidly delivering 10-15 IUs of insulin to the patient could adversely affect patient health.

To prevent such undesirable outcomes, and for user convenience in other situations involving the choice between a variety of disposable assemblies (such as disposable assemblies with reservoirs containing different medicaments, different concentrations of a medicament, and/or varying amounts of medicaments), at least some of the present disposable assemblies may be provided with a baseplate identification device and at least some of the present disposable assemblies may be provided with structure that cooperate with a baseplate identification device in such a manner that the durable assembly microprocessor/controller can make a "baseplate type" determination. Exemplary baseplate identification instrumentalities and methodologies may be as described in aforementioned U.S. patent publication numbers 2012/0078170, 2012/0184907, and 2013/0138078. In addition, baseplate identification may be performed mechanically. For instance, a pin or rib may prevent attachment of certain disposable assemblies with certain durable assemblies. Additionally or alternative, certain durable assemblies will simply not function with certain disposable assemblies.

Alternatively, the patient or a clinician may program the system, such as via a remote control, to indicate the type of disposable assembly attached. In a manner such as this, a patient can access a variety of medicaments for use with a single durable assembly.

Once the "baseplate type" determination is made (e.g., "patch pump" disposable assembly 300 versus a "pocket pump" with infusion set 503 attached), the durable assembly will proceed in a manner, or mode of operation, that is appropriate for the attached disposable assembly. For example, if "patch pump" disposable assembly 300 is detected, the durable assembly controller will not include priming as part of the delivery process and, in some implementations, will prevent the user from manually implementing a priming procedure. If, on the other hand, a "pocket pump" disposable assembly is detected, then the delivery process may include appropriate priming of the infusion set tube.

Whether configured as a "pocket pump" or a "patch pump," the system may be configured to provide basal delivery of medicament in accordance with a delivery profile provided by a physician by way of a clinician's programming unit. For example, the system may include a program that stores a number of delivery profiles (e.g., delivery profiles associated with a 24-hour delivery cycle, delivery profiles for particular situations such as sleep or illness, and the like). Each delivery profile specifies multiple doses (or pump "operations") over time, e.g., a particular number of doses at particular times or a particular number of doses per unit time. In some implementations, a dose may be the volume associated with the minimum controllable displacement of the plunger 335. The system may also be configured to provide bolus delivery in response to an instruction from a patient remote control 1000 (FIG. 2A). A bolus instruction may come in response to a high glucose level measurement in the case of a diabetic patient, an increase in pain level in the case of a pain management patient, or some other symptom. The system may also be configured to perform other functions, such as ending medicament delivery in response to instructions from patient remote control 1000.

The present infusion pumps may be used in conjunction with a wide variety of remote controls. Such remote controls may be used to, for example, allow the user to transmit instructions to the durable assembly 200 or facilitate communication between durable assembly 200 and the user (e.g., an alarm condition message or other message concerning the conditions of system 100). An exemplary remote control 1000 (FIG. 2A) may be configured to facilitate one, some, or all of the following operations: (1) turning the remote control 1000 on or off, (2) associating (or "assigning") the remote control 1000 to the durable assembly 20, (3) obtaining status information such as medicament level, battery charge level, and/or alarm conditions, (4) silencing the durable assembly alarm, (5) selecting options that may be associated with the durable assembly alarm such as type of alarm (audible, palpable, and/or visible) and strength/volume of alarm, (6) connecting remote control 1000 to a computer to, for example, update remote control or durable assembly firmware, load and delete delivery profiles stored in the durable assembly or remote control, and otherwise reprogram the durable assembly or remote control, (7) selecting medicament options such as medicament concentrations, (8) selecting and initiating a stored medicament delivery profile, (9) increasing and decreasing medicament dose rate, and/or (10) pausing a dispensing operation. A user may pause delivery in order to remove or replace a patient applied structure (e.g., a disposable assembly), adjust for a current or anticipated changed body condition (e.g., low glucose, vigorous exercise), follow a physician's suggestion, or disconnect the durable assembly from the body for any other reason.

The exemplary remote control 1000 (FIG. 2A) may be configured to generate an indicator, based on information from a microprocessor 223 for durable assembly 200, that is indicative of, for instance, the amount of time remaining in the current dispensing program, the amount of time until the next disposable assembly replacement, etc. The indicator may be audible, visible, palpable, or combinations thereof. A time remaining indicator may be useful for a variety of reasons. For example, knowledge of the time remaining prior to next disposable assembly replacement allows the patient to determine, based at least in part on the current time of day and upcoming events (e.g., travel or sleep), whether or not it would be more convenient to replace the disposable assembly at a time prior to the end of the dispensing program.

As described above, parts of the present systems may be considered the reusable parts, while other parts may be considered the disposable parts. In the illustrated embodiments, the durable assembly 200, which may include structures such as microprocessor 223 and coil assembly 224, is reusable, while exemplary disposable assemblies 300, which may include structures such as a motor rotor 331 and reservoir 336 on a baseplate 350, are disposable.

With respect to dimensions, some embodiments of the exemplary infusion pump system 100 may have the following dimensions: length dimensions of 35 mm +/- 1.0 mm, 35 mm +/- 0.10 mm, or 35 mm +/- 5.0 mm; width dimensions of 30 mm +/- 1.0 mm, 30 mm +/- 0.10 mm, or 30 mm +/- 5 mm; and overall thickness or height dimensions of 8.5 mm +/- 1.0 mm, 8.5 mm +/- 2 mm, or 8.5 mm +/- 0.10 mm. Suitable housing materials include, but are not limited to, plastic or other materials having a modulus of elasticity of 0.2-1.0 million psi.

Exemplary durable assembly microprocessors and associated circuitry; rechargeable batteries and associated battery rechargers and recharging methods; battery and recharging management; temperature sensors; and exemplary alarms and alarm conditions are described in more detail in aforementioned U.S. patent publication numbers 2012/0078170, 2012/0184907, and 2013/0138078.

The reservoirs may also be, but are not required to be, prefilled. Prefilled reservoirs are advantageous for a variety of reasons. By way of example, but not limitation, some users prefer to avoid reservoir filling procedures because they are inconvenient and tend to involve needles. User-based refilling also increases the likelihood that air bubbles will be introduced into the reservoir, while prefilling by the manufacturer of the reservoir and/or the medicament can be accomplished without any substantial introduction of air bubbles using, for example, a vacuum filling procedure. Nevertheless, user-filled reservoirs may be employed in some instances. A variety of exemplary medicament reservoirs, including those that include pressure sensors (such as for sensing occlusion) and other sensors, are described in more detail in aforementioned U.S. patent publication numbers 2012/0078170, 2012/0184907, and 2013/0138078.

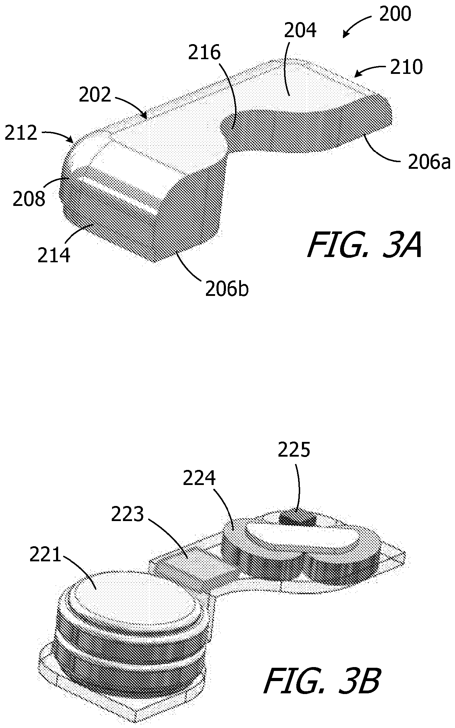

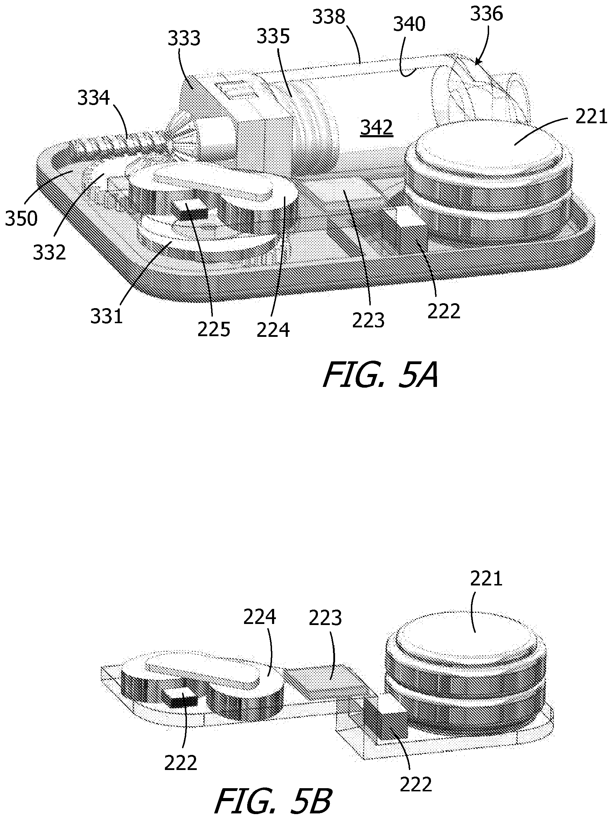

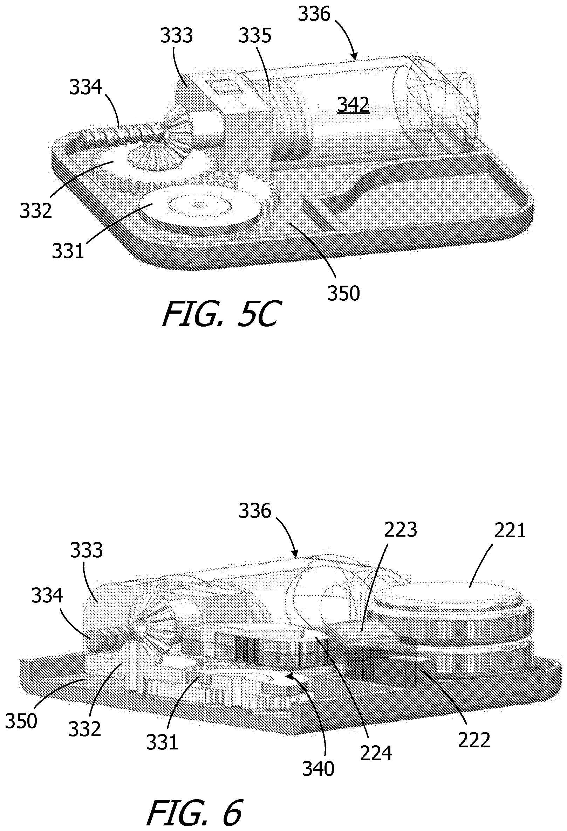

Turning now to FIGS. 3A and 3B, an exemplary durable assembly 200 may include a power source such as one or more batteries 221, temporary power storage such as one or more capacitors 222 (see FIGS. 2 and 5B), a controller such as microprocessor 223, a coil assembly 224, and a hall effect sensor 225. Those of skill in the art will appreciate that including the motor's coil assembly 224 and all other electronics within the durable assembly 200 reduces the cost and complexity of disposable assembly 300. In addition, the microprocessor 223 provides flexibility to include features such as user data storage, programs, programmability, adjustability, a display, buttons, wireless communication protocols, or the like to the pump 100. Durable assembly 200 may also be molded with locking features that snap onto the disposable assembly 300, but that also allow removal of the durable assembly 200 from the disposable assembly 300 either while the disposable assembly remains in place on the patient (after medicament delivery has been paused), or after the entire system has been removed from the patient.

The power source may be one or more commercially available batteries, such as a commercially available zinc-air battery or lithium polymer battery. The batteries may be selected to have sufficient capacity to operate the system for certain delivery amounts or delivery times, such as for over 400 units of delivered insulin. The optional power storage may be one or more commercially available capacitors or super-capacitors or other temporary storage device(s).

Turning now to FIGS. 4A and 4B, an exemplary disposable assembly 300 may include baseplate 350 and components such as a reservoir 336, a plunger 335 within the reservoir and connected to lead screw 334, and a magnetic motor rotor 331 mechanically attached through gear train 332 to affect rotation of the lead screw drive gear 333, which causes translation of the lead screw 334 and plunger 335 within reservoir 336. The cover 302 is positioned over these components in the illustrated embodiment. The exemplary baseplate 350 includes an adhesive backing for attachment to the patient with a removable adhesive cover. The baseplate 350 may also be molded with baseplate locking features that snap onto the durable assembly 200 (such as magnets molded into the housings of each assembly), and that also allows removal of the durable assembly 200 from the disposable assembly 300.

Referring to FIG. 2 and 4B, the exemplary reservoir 336 includes a barrel 338 with an inner surface 340 defining a fluid storage volume 342 and an oval cross-section, but other shapes are possible. A plunger 335 with a matching cross-sectional shape fits within the barrel and carries a fluid seal such as, but not limited to, o-rings, to seal the medicament within the storage volume 342. The exemplary plunger 335 is formed from rubber and includes three o-ring seals. The reservoir 336 includes a connector 501 that may be used for filling reservoir 336, or for attaching a cannula for "patch-pump" type configurations, or for connecting (potentially via an appropriate adapter(s)) an infusion set for "pocket-pump" type configurations. The plunger 335 moves within the barrel 338 to vary the volume of medicament within the storage volume 342. Reservoir 336 may be, for instance, prefilled (or user-filled) with U-500 insulin in various volumes to suit the patient use profile. In other instances, lower concentrations of insulin, such as U-100 insulin and U-200 insulin, may be employed. A plug may be inserted in the connector 501 to maintain a sterile environment until use. The patient would remove the plug prior to use, in those instances.

Additional exemplary baseplates for use with the disposable assemblies of the present inventions, as well as exemplary cannula designs, fluidic connection between a medicament reservoir and the cannula, cooperation between the cannula and disposable assemblies (for instance, to prevent axial movement of the cannula relative to the baseplate and patient), attachment of an infusion set to the reservoir of the disposable assembly, configurations and uses of a non-delivery baseplate, arrangements and structures for attaching disposable and durable assemblies, skin adhesive designs, and various occlusion sensors, may be as described in U.S. patent application serial number 12/890,207, filed September 24, 2010 and corresponding U.S. patent publication number 2012/0078170, as well as aforementioned U.S. patent publication numbers 2012/0184907 and 2013/0138078.

Turning now to FIGS. 5A-5C and the illustrated two-piece motor, the motor's coil assembly 224 (and a Hall effect sensor 225) of the durable assembly 200 are positioned above the magnetic motor rotor 331 that is part of the disposable assembly 300. An exemplary multi-pole motor rotor 331 may be disc-shaped and have a 9.8 mm outer diameter, 5.2 mm inner diameter, and 0.8 mm thickness. Another example motor rotor may have an 11 mm outer diameter, 5 mm inner diameter, and 1.2 mm thickness. Multi-pole motor rotors of this type typically cost less than 5 cents per piece, helping control the total cost of disposable assembly 200. The motor rotor 331 is also parallel to the baseplate 350, i.e., the motor rotor axis of rotation is perpendicular to the baseplate, in the illustrated embodiment. The microprocessor 223 directs rotation of motor rotor 331 by sequentially energizing the coils of motor coil assembly 224 to create an electromagnetic torque coupling between the motor coil assembly 224 and the motor rotor 331. The position/orientation of the rotor's poles relative to the rotating magnetic field generator (coil assembly 224) is measured by back EMF, a rotary encoder, a hall effect sensor 225 (FIG. 5A), or the like. For instance, a Hall effect sensor mounted on the coil windings may be used to supply the microprocessor a count, a tachometer signal, or rotor position, allowing low-cost closed-loop control of the rotor speed. Brushless motors of this type are typically 85-90% or more efficient, and run very cool. While there may be variations in construction, the face-to-face stator coils and flat rotor plate shown in FIGS. 5A-5C provide a compact design. In addition, more coils and/or Hall effect sensors may be used.

As can best be seen in FIG. 6, between the motor coil assembly 224 and motor rotor 331 is a gap 240. Some or all of the gap 240 may be defined by (and occupied by) portions of the housing 202 and the cover 302, i.e., the housing bottom wall 206a and the cover top wall 304b in the illustrated implementation. In other implementations, the gap 240 between the between the motor coil assembly 224 and motor rotor 331 may be occupied by only a portion of the durable assembly housing, or only a portion of the disposably assembly cover, or no structure at all and may simply be an air gap. The size of the gap, which is defined by the distance between the motor coil assembly 224 and the motor rotor 331, is typically about 0.5 mm to 2.0 mm. As such, there is no gear engagement or other mechanical connection between the durable assembly 200 and disposable assembly 300. And as described earlier, all electronics may be positioned within the durable assembly 200, with the energy needed by the disposable assembly 300 transferred by electromagnetic torque coupling, which is a coupling without direct mechanical coupling or electrical contact from the durable assembly 200. This exemplary design affords the additional advantage of being relatively simple to make waterproof, or at least water resistant.

As described above, rotation of motor rotor 331 drives gear train 332, causing rotation of lead screw drive gear 333, which in turn affects translation of the lead screw 334 and plunger 335, which is attached to lead screw 334. In this manner, electromagnetically generated torque is created when electromagnetic energy supplied by durable assembly 200 is transformed into mechanical forces within the disposable assembly 300 that advance plunger 335. A ratchet (not shown) or other similar device may be used to prevent back drive of gear train 332. As plunger 335 is driven through reservoir 336, medicament is dispensed precisely, corresponding to the precision movements of the gears and motor rotor. With the entire gear train, lead screw drive gear, lead screw, and plunger all permanently contained in the disposable assembly 300, there is no need to retract any plunger components into the durable assembly 200 prior to separation from the disposable assembly 300. As a result, a further advantage of this exemplary design is greatly reduced energy consumption, which allows use of, for instance, a primary battery(ies) as a power source.

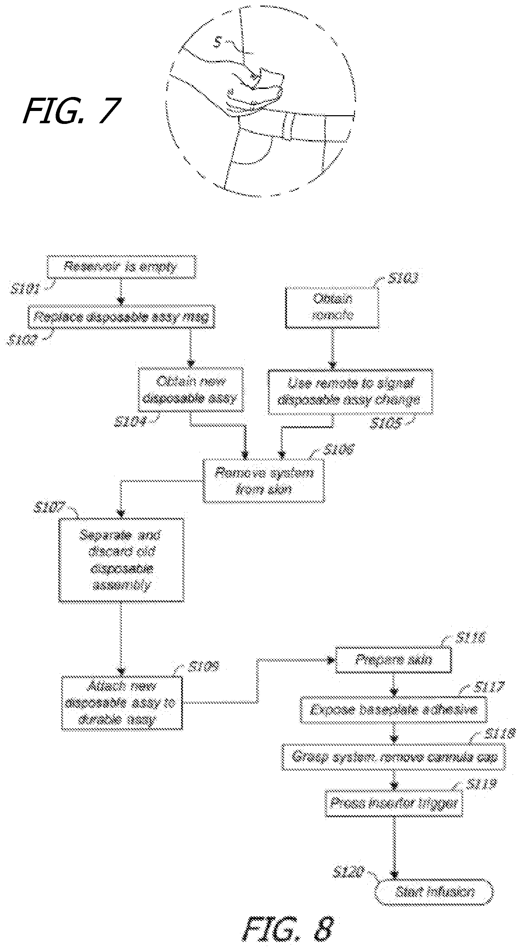

Use of an exemplary system 100 will now be described. At the most basic level, a patient's use of the exemplary infusion pump systems (e.g., system 100 in FIGS. 1A-2B) involves obtaining a new disposable assembly 300, connecting the disposable assembly to the durable assembly 200, peeling the liner from the baseplate adhesive layer, gaining subcutaneous access, and initiating a medicament delivery operation. In some instances, use may involve additional steps such as attaching a cannula to connector 501 of the disposable assembly and removing a cannula cap, if necessary. Various aspects of the basic operation of the present systems are described below. Operation of a system does not necessarily require all of the steps each time the system is deployed, and the order of some of the steps may be changed. Operation is also discussed below, in the exemplary context of the above-described durable assembly 200 and disposable assembly 300 used as a patch pump, through the use of a flow chart (FIG. 8). The discussion is, however, equally applicable to other patch pump implementations, as well as to pocket pump implementations with minor variations. Also, unless otherwise indicated, the actions and determinations performed by the durable assembly 200 are controlled by the durable assembly microprocessor and further references to the controller are limited in the interest of brevity.

Referring to FIG. 8, use of the present systems may involve removal of a disposable assembly from a durable assembly and the replacement of the disposable assembly. This may occur when the medicament reservoir is empty (as described in more detail in U.S. patent application serial number 12/890,207 and corresponding U.S. patent publication number 2012/0078170) (Step S101) and a "replace disposable assembly" message or alert is presented (Step S102), or when the durable assembly controller receives a user-initiated "replace disposable assembly" signal from a remote control 1000 (Step S103). The user may desire to replace a disposable assembly before the medicament reservoir is empty for a variety of reasons such as, for example, to accommodate the user's sleep or travel schedule, when the medicament exhibits a loss of effectiveness, when a dispensing problem arises, or due to a prescribed change in medicament.

The user may then obtain, possibly from storage in a refrigerator depending on medicament requirements, a new disposable assembly 300 (Step S104). The durable assembly 200 and disposable assembly 300 may then be removed from the skin, separated, and the disposable assembly 300 discarded (Steps S106 and S107).

Next, the new disposable assembly 300 may be attached to the durable assembly 200 (Step S109). The user should clean the skin surface S onto which the baseplate 350 of disposable assembly 300 will be adhered (FIG. 7, and Step S116 of FIG. 8). Then the user peels off the baseplate adhesive liner to expose the baseplate adhesive layer (Step S117) and removes cannula cap (when present) (Step S118). In the exemplary use of FIG. 8, the disposable assembly 30 is supplied with a cannula pre-attached to the connector 501. In other embodiments, a cannula inserter may be attached to the system, which may be triggered to insert the cannula after the system in placed against the skin. Exemplary inserters are described in U.S. patent publication number 2013/0138078.

Returning to the steps in FIG. 8, the system 100 including durable assembly 200 and disposable assembly 300 may be positioned over a suitable body location and pressed gently to adhere the adhesive layer to the skin surface S (Step S119). Finally, if necessary, the remote control 1000 may be used to initiate a particular medicament delivery operation (Step S120). The delivery operation may follow a predetermined delivery profile (e.g. a particular basal rate, a series of time-spaced bolus deliveries, or some combination thereof) that is equated to motor rotor rotations, at particular rates and times, required to deliver medicament in accordance with the profile. Alternatively, the profile may be input by the user with the remote control 1000 and stored by the durable assembly microprocessor. For example, the remote control may store a number of different delivery profiles and bolus deliveries from which the patient can choose. Such profiles may correspond to, for example and depending on the medicament, days where vigorous exercise is expected, days where it is not, incidences of increased pain, etc. Alternatively, or in addition, the profile stored in the durable assembly microprocessor may be set by a clinician's programming unit. In such a case, as in the case of different disposable assemblies 300 provided with different specified delivery rates, a remote control may not be needed to initiate, e.g., basal delivery.

The discussion above is also applicable to use of the "pocket pump" system as shown in FIG 2B. Minor variations in the above-described procedure include, for example, use of an infusion set 503 instead of a cannula, attaching the infusion set to connector 501, potentially via an adapter (which may vary with the type of infusion set 503), and priming of the infusion set tube.

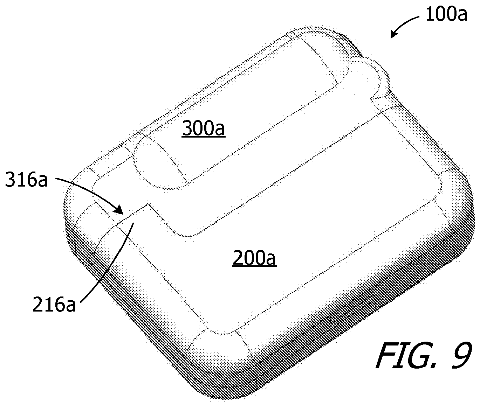

Another exemplary ambulatory infusion system, which is generally represented by reference numeral 100a in FIG. 9, includes a durable assembly 200a and a disposable assembly 300a. System 100a is substantially similar to system 100. Here, however, the intersection of the top walls is primarily linear. Additionally, the disposable assembly 300a has a recess 316a which mates with a corresponding projection 216a on the durable assembly 200a. The projection 216a and recess 316a are located at the outer perimeter of the assembled system 100a.

Various methodologies are presented here in the context of the exemplary structures described in the preceding sections, and illustrated in the various figures, for the purpose of explanation only. Although the present methodologies may employ the structures described above, they are not limited thereto. Additionally, the durable assembly may provide audible, visible and/or tactile notifications. A remote control may also provide audible, visible and/or tactile notifications as an alternative to, or in addition to, any notifications provided by a durable assembly. Additionally, embodiments of the present inventions may incorporate any one, combinations of less than all, or all of the methodologies or devices referenced above.

Although the inventions disclosed herein have been described in terms of the preferred embodiments above, numerous modifications and/or additions to the above-described preferred embodiments would be readily apparent to one skilled in the art. It is intended that the scope of the present inventions extends to all such modifications and/or additions and that the scope of the present inventions is limited solely by the claims set forth below or later added.

Finally, with respect to terminology that may be used herein, whether in the description or the claims, the following should be noted. The terms "comprising," "including," "carrying," "having," "containing," "involving," and the like are open-ended and mean "including but not limited to." Ordinal terms such as "first", "second", "third," do not, in and of themselves, connote any priority, precedence, or order of one element over another or temporal order in which steps of a method are performed. Instead, such terms are merely labels to distinguish one element having a certain name from another element having a same name (but for the ordinal term) to distinguish the elements. "And/or" means that the listed items are alternatives, but the alternatives also include any combination of the listed items. The terms "approximately," "about," "substantially" and "generally" allow for a certain amount of variation from any exact dimensions, measurements, and arrangements, and should be understood within the context of the description and operation of the invention as disclosed herein. Terms such as "top," "bottom," "above," and "below" are terms of convenience that denote the spatial relationships of parts relative to each other rather than to any specific spatial or gravitational orientation. Thus, the terms are intended to encompass an assembly of component parts regardless of whether the assembly is oriented in the particular orientation shown in the drawings and described in the specification, upside down from that orientation, or any other rotational variation therefrom.

## Claims

1. An infusion pump system, comprising:
a disposable assembly (300) including a medicament reservoir (336) and a multi-pole motor rotor (331); and
a durable assembly (200) including an energy supply (221), a motor coil (224), a sensor (225) configured to sense rotational position of the multi-pole motor rotor (331), and a processor (223), operably connected to the energy supply (221), motor coil (224) and sensor (225), configured to control power to the motor coil (224) based on the sensed rotational position of the multi-pole motor rotor (331);
wherein the durable assembly (200) and the disposable assembly (300) are configured to be attached to one another in such a manner that energization of the motor coil (224) causes the multi-pole motor rotor (331) to rotate without direct mechanical coupling or electrical connection.

2. An infusion pump system as claimed in claim 1, wherein
the energy is transferred through electromagnetic torque coupling.

3. An infusion pump system as claimed in claim 1, wherein
the motor coil (224) and the multi-pole motor rotor (331) are separated by a gap (240).

4. An infusion pump system as claimed in claim 3, wherein
the durable assembly (200) includes a housing (202) and a portion of the housing is located in the gap (240).

5. An infusion pump system as claimed in claim 3, wherein
the disposable assembly (300) includes a baseplate (350) and a cover (302) and a portion of the cover is located in the gap (240).

6. An infusion pump system as claimed in claim 3, wherein
the gap (240) is 0.5 mm to 2.0 mm.

7. An infusion pump system as claimed in claim 1, wherein
the medicament reservoir (336) includes a barrel (338) and a plunger (335) within the barrel (338);
the multi-pole motor rotor (331) is mechanically coupled to the plunger (335) such that rotation of the multi-pole motor rotor (331) results in movement of the plunger (335); and
the processor (223) controls plunger speed and plunger position by controlling power to the motor coil (224).

8. An infusion pump system as claimed in claim 1, wherein
the energy supply (221) includes a battery.

9. An infusion pump system as claimed in claim 8, wherein
the battery comprises either one or more primary cell batteries, or one or more rechargeable batteries.

10. An infusion pump system as claimed in claim 1, wherein
the disposable assembly (300) includes a baseplate (350); and
the multi-pole motor rotor (331) defines an axis of rotation that is perpendicular to the baseplate (350).

11. A method, comprising the step of:
after an infusion pump disposable assembly (200) including a medicament reservoir (336) and a multi-pole motor rotor (331) has been secured to an infusion pump durable assembly (300), with an energy supply (221), a motor coil (224), and a sensor (225), such that energy from the energy supply (221) is transferred to the disposable assembly (200) without direct mechanical coupling or electrical connection and transformed into mechanical forces that move a plunger (335) within the reservoir (336) and the sensor (225) senses the rotational position of the multi-pole motor rotor (331), closed-loop controlling rotational speed of the multi-pole motor rotor (331) with a controller (223) based on output from the sensor (225).

12. A method as claimed in claim 11, wherein
the energy supply (221) includes one or more batteries.

13. A method as claimed in claim 11, wherein
the infusion pump durable assembly (300) and the infusion pump disposable assembly (200) are configured such that, when secured to one another, there is a gap (240) between the motor coil (224) and the multi-pole motor rotor (331).

14. A method as claimed in claim 13, wherein
a portion of at least one of the durable assembly (300) and the disposable assembly (200) is located within the gap (240).

15. A method as claimed in claim 11, wherein
energy is transferred from the energy supply to the disposable assembly (200) through electromagnetic torque coupling.

## Patentansprüche

1. Infusionspumpensystem, umfassend:
eine Einweg-Anordnung (300) mit einem Arzneimittelbehälter (336) und einem mehrpoligen Motorrotor (331); und
eine langlebige Anordnung (200) mit einer Energieversorgung (221), einer Motorspule (224), einem Sensor (225), der so konfiguriert ist, dass er die Drehposition des mehrpoligen Motorrotors (331) erfasst, und einem Prozessor (223), der betriebsfähig mit der Energieversorgung (221), der Motorspule (224) und dem Sensor (225) verbunden und so konfiguriert ist, dass er die Leistung für die Motorspule (224) auf der Grundlage der erfassten Drehposition des mehrpoligen Motorrotors (331) steuert;
wobei die langlebige Anordnung (200) und die Einweg-Anordnung (300) so konfiguriert sind, dass sie so aneinander befestigt werden können, dass die Erregung der Motorspule (224) bewirkt, dass sich der mehrpolige Motorrotor (331) ohne direkte mechanische Kopplung oder elektrische Verbindung dreht.

2. Infusionspumpensystem nach Anspruch 1, wobei die Energie durch elektromagnetische Drehmomentkupplung übertragen wird.

3. Infusionspumpensystem nach Anspruch 1, wobei die Motorspule (224) und der mehrpolige Motorrotor (331) durch einen Spalt (240) getrennt sind.

4. Infusionspumpensystem nach Anspruch 3, wobei die langlebige Anordnung (200) ein Gehäuse (202) aufweist und ein Teil des Gehäuses in dem Spalt (240) angeordnet ist.

5. Infusionspumpensystem nach Anspruch 3, wobei die Einweg-Anordnung (300) eine Grundplatte (350) und eine Abdeckung (302) aufweist und wobei ein Teil der Abdeckung in dem Spalt (240) angeordnet ist.

6. Infusionspumpensystem nach Anspruch 3, wobei der Spalt (240) 0,5 mm bis 2,0 mm beträgt.

7. Infusionspumpensystem nach Anspruch 1, wobei der Arzneimittelbehälter (336) einen Zylinder (338) und einen Kolben (335) innerhalb des Zylinders (338) aufweist;
wobei der mehrpolige Motorrotor (331) mechanisch derart mit dem Kolben (335) gekoppelt ist, dass die Drehung des mehrpoligen Motorrotors (331) eine Bewegung des Kolbens (335) bewirkt; und
wobei der Prozessor (223) die Kolbengeschwindigkeit und Kolbenposition durch Steuerung der Leistung zur Motorspule (224) steuert.

8. Infusionspumpensystem nach Anspruch 1, wobei die Energieversorgung (221) eine Batterie umfasst.

9. Infusionspumpensystem nach Anspruch 8, wobei die Batterie entweder eine oder mehrere Primärzellenbatterien oder eine oder mehrere wiederaufladbare Batterien umfasst.

10. Infusionspumpensystem nach Anspruch 1, wobei die Einweg-Anordnung (300) eine Grundplatte (350) umfasst; und
wobei der mehrpolige Motorrotor (331) eine Drehachse definiert, die senkrecht zur Grundplatte (350) verläuft.

11. Verfahren, umfassend den folgenden Schritt:
nachdem eine Einweg-Anordnung (200) einer Infusionspumpe, die einen Arzneimittelbehälter (336) und einen mehrpoligen Motorrotor (331) umfasst, an einer langlebigen Anordnung (300) einer Infusionspumpe mit einer Energieversorgung (221), einer Motorspule (224) und einem Sensor (225) derart befestigt worden ist, dass Energie von der Energieversorgung (221) ohne direkte mechanische Kopplung oder elektrische Verbindung auf die Einweg-Anordnung (200) übertragen und in mechanische Kräfte umgewandelt wird, die einen Kolben (335) innerhalb des Behälters (336) bewegen, und der Sensor (225) die Drehposition des mehrpoligen Motorrotors (331) erfasst, wobei die Drehgeschwindigkeit des mehrpoligen Motorrotors (331) mit einem Regler (223) auf der Grundlage der Ausgabe des Sensors (225) in einem geschlossenen Regelkreis geregelt wird.

12. Verfahren nach Anspruch 11, wobei die Energieversorgung (221) eine oder mehrere Batterien umfasst.

13. Verfahren nach Anspruch 11, wobei die langlebige Anordnung (300) der Infusionspumpe und die Einweg-Anordnung (200) der Infusionspumpe derart konfiguriert sind, dass, wenn sie aneinander befestigt sind, ein Spalt (240) zwischen der Motorspule (224) und dem mehrpoligen Motorrotor (331) vorhanden ist.

14. Verfahren nach Anspruch 13, wobei ein Teil der langlebigen Anordnung (300) und/oder der Einweg-Anordnung (200) innerhalb des Spalts (240) angeordnet ist.

15. Verfahren nach Anspruch 11, wobei Energie von der Energieversorgung auf die Einweg-Anordnung (200) durch elektromagnetische Drehmomentkupplung übertragen wird.

## Revendications

1. Système de pompe à perfusion, comprenant :
un ensemble jetable (300) comportant un réservoir de médicament (336) et un rotor de moteur multipolaire (331) ; et
un ensemble durable (200) comportant une alimentation en énergie (221), une bobine de moteur (224), un capteur (225) configuré pour détecter la position de rotation du rotor de moteur multipolaire (331), et un processeur (223), fonctionnellement connecté à l'alimentation en énergie (221), à la bobine de moteur (224) et au capteur (225), configuré pour commander l'alimentation de la bobine de moteur (224) sur la base de la position de rotation détectée du rotor de moteur multipolaire (331) ;
l'ensemble durable (200) et l'ensemble jetable (300) étant configurés pour être fixés l'un à l'autre de telle manière que l'excitation de la bobine de moteur (224) provoque la rotation du rotor de moteur multipolaire (331) sans accouplage mécanique direct ni connexion électrique.

2. Système de pompe à perfusion selon la revendication 1, l'énergie étant transférée par accouplage de couple électromagnétique.

3. Système de pompe à perfusion selon la revendication 1, la bobine de moteur (224) et le rotor de moteur multipolaire (331) étant séparés par un espace (240).

4. Système de pompe à perfusion selon la revendication 3, l'ensemble durable (200) comportant un boîtier (202) et une partie du boîtier étant située dans l'espace (240).

5. Système de pompe à perfusion selon la revendication 3, l'ensemble jetable (300) comportant une plaque de base (350) et un couvercle (302) et une partie du couvercle étant située dans l'espace (240).

6. Système de pompe à perfusion selon la revendication 3, l'espace (240) étant de 0,5 mm à 2,0 mm.

7. Système de pompe à perfusion selon la revendication 1, le réservoir de médicament (336) comportant un cylindre (338) et un piston (335) à l'intérieur du cylindre (338) ;
le rotor de moteur multipolaire (331) est accouplé mécaniquement au piston (335) de sorte que la rotation du rotor de moteur multipolaire (331) entraîne le mouvement du piston (335) ; et
le processeur (223) commande la vitesse du piston et la position du piston en commandant l'alimentation de la bobine de moteur (224).

8. Système de pompe à perfusion selon la revendication 1, l'alimentation en énergie (221) comportant une batterie.

9. Système de pompe à perfusion selon la revendication 8, la batterie comprenant soit une ou plusieurs batteries de cellules primaires, soit une ou plusieurs batteries rechargeables.

10. Système de pompe à perfusion selon la revendication 1, l'ensemble jetable (300) comportant une plaque de base (350) ; et
le rotor de moteur multipolaire (331) définissant un axe de rotation qui est perpendiculaire à la plaque de base (350).

11. Procédé comprenant les étapes suivantes :
après qu'un ensemble jetable de pompe à perfusion (200) comportant un réservoir de médicament (336) et un rotor de moteur multipolaire (331) a été fixé à un ensemble durable de pompe à perfusion (300), avec une alimentation en énergie (221), une bobine de moteur (224), et un capteur (225), de telle sorte que l'énergie provenant de l'alimentation en énergie (221) est transférée à l'ensemble jetable (200) sans accouplage mécanique direct ni connexion électrique et transformée en forces mécaniques qui déplacent un piston (335) à l'intérieur du réservoir (336) et le capteur (225) détecte la position de rotation du rotor de moteur multipolaire (331), en commandant en boucle fermée la vitesse de rotation du rotor de moteur multipolaire (331) avec un dispositif de commande (223) sur la base de la sortie du capteur (225).

12. Procédé selon la revendication 11, l'alimentation en énergie (221) comportant une ou plusieurs batteries.

13. Procédé selon la revendication 11, l'ensemble durable de pompe à perfusion (300) et l'ensemble jetable de pompe à perfusion (200) étant configurés de telle sorte que, lorsqu'ils sont fixés l'un à l'autre, il existe un espace (240) entre la bobine de moteur (224) et le rotor du moteur multipolaire (331).

14. Procédé selon la revendication 13, une partie d'au moins l'un de l'ensemble durable (300) et de l'ensemble jetable (200) étant située à l'intérieur de l'espace (240).

15. Procédé selon la revendication 11, l'énergie étant transférée de l'alimentation en énergie à l'ensemble jetable (200) par accouplage de couple électromagnétique.
